# DEMANDE DE BREVET EUROPEEN

(11) **EP 1 550 433 A1**
(43) Date de publication de la demande: **06.07.2005**
(21) Numéro de dépôt: 04293075.0
(22) Date de dépôt: 22.12.2004
(51) Int. Cl.: A61K 7/06, A61K 31/575

(54) **Utilisation d'ecdystéroides pour la préparation de compositions dermatologiques ou cosmétiques anti-chute de cheveux**

(30) Priorité: 22.12.2003 FR 0315157
(71) Demandeur: L'OREAL, 75008 Paris (FR)
(72) Inventeur: Bernard, Bruno, 92200 Neuilly sur Seine (FR); Gautier, Brigitte, 91940 Les Ulis (FR)
(74) Mandataire: Dossmann, Gérard

(57) **Abrégé**

La présente invention concerne l'utilisation d'au moins un ecdystéroïde pour la préparation de compositions cosmétiques ou dermatologiques destinées à empêcher la chute des cheveux.

## Description

La présente invention concerne l'utilisation d'au moins un ecdystéroïde pour la préparation de compositions cosmétiques ou dermatologiques destinées à empêcher la chute des cheveux.

Les ecdystéroïdes sont un groupe de 2,3,14-trihydroxy-Δ-7-6-kétostéroïdes. Ces molécules sont bien connues dans la littérature et citées dans le Merck Index, 10^{e} édition, 1983, page 505, n°3 470.

On sait que les ecdystéroïdes, et en particulier l'ecdystérone (ou β-ecdysone) jouent un rôle important aussi bien dans le règne animal chez les insectes, où l'ecdystérone intervient en particulier dans la métamorphose, que dans le règne végétal où ces substances semblent jouer un rôle dans la floraison.

Il a été récemment découvert que les ecdystéroïdes régulent la différenciation des kératinocytes de l'épiderme. Au niveau du follicule pileux, ces hormones régulent voire augmentent la synthèse de la kératine, constituant principal de la tige pilaire (US 5,609,873). L'homme de l'art pourra se reporter à l'article de Eckert et Rorke (1989, *Environment Health Perspective,* **80** :109-116).

L'ecdystérone, en particulier, a été décrite comme agent permettant d'améliorer l'apparence de la peau (CN 86 1 06791A) et des cheveux (US 5,609,873), ainsi que la qualité constitutive de la fibre capillaire (FR 93 10138). De plus, l'ecdystérone est connue pour stimuler la repousse du poil dans le modèle murin C3H (H. Ishida *et al.,* 1999, *Biol. Pharma. Bull.* **22** :1189-1192).

L'ecdystérone, hormone de mue chez l'insecte, est connue pour modifier l'expression d'un certain nombre de gènes chez l'insecte via un récepteur nucléaire spécifique. Compte-tenu de l'analogie structurale entre l'ecdystérone et les stéroïdes tels que les androgènes, estrogènes, etc... dont on sait qu'ils modifient la croissance du cheveu, la demanderesse a eu l'idée d'évaluer l'effet de l'ecdystérone sur la croissance et la survie du follicule pileux.

La demanderesse a ainsi découvert de façon surprenante et inattendue, que l'ecdystérone était capable de favoriser la survie du follicule humain *in vitro* et de maintenir sans les stimuler d'une part l'activité mitotique des cellules de la matrice du follicule pileux humain, et d'autre part, les programmes de différenciation de la gaine externe et de la gaine interne du follicule, tout en assurant l'intégrité de la tige pilaire.

L'invention a ainsi pour objet l'utilisation d'ecdystéroïdes dans la préparation d'une composition à usage cosmétique ou dermatologique.

L'invention a également pour objet un procédé de traitement cosmétique comportant l'utilisation de cette composition pour empêcher la chute de cheveux.

D'autres objets de la présente demande ressortiront à la lecture de la description et des exemples qui suivent.

La présente invention a ainsi pour objet l'utilisation d'au moins un ecdystéroïde ou d'au moins un analogue d'ecdystéroïde ou d'au moins un dérivé d'ecdystéroïde pour la préparation d'une composition cosmétique ou dermatologique destinée à empêcher la chute de cheveux.

Par analogue d'ecdystéroïde, on entend des stéroïdes généralement caractérisés par une jonction cis des cycles A et B, un chromophore 7-en-6-one, et un groupe 14α-hydroxy. Ces analogues possèdent en général un squelette de type cholestérol (C27) ou phytostérol (C28, C29) (C. Blais *et al,* 1996, *Biochem. J.* 320 : 413-419). Actuellement, plus de 250 analogues structuraux ont été identifiés (Laffont R. et Wilson ID, 1996, The ecdysone handbook, 2nd edition, The Chromatographic Society, Nottingham, U.K., pp 525 ; voir site internet http://ecdybase.org).

Des dérivés d'ecdystéroïdes peuvent être des produits d'oxydation des ecdystéroïdes ou de ces analogues, de réduction ou de conjugaison. De préférence, les dérivés d'ecdystéroïdes sont des dérivés acylés, hydroxylés ou désoxylés.

De préférence, les ecdystéroïdes, analogues d'ecdystéroïdes ou dérivés d'ecdystéroïdes sont choisies parmi l'ecdystérone, l'ecdysone, la muristérone A, les ponastérones A, B et C, l'inokostérone, des phytoecdystéroïdes telles que les limnanthéosides A et B (J. Preston-Mafham & L. Dinan, 2002, Z. Naturforsch. 57c : 144-152), les ajugastérones B et C, la turkestérone, la podécydsone, la stachystérone, ou les ecdystéroïdes 7, 9 (11)-dien-6-ones comme la kaladastérone, la dacryhainanstérone ou la 25-hydroxydacryhainanstérone (P.C. Bourne *et al.,* 2002, J. *Insect Sci.***2** : 11).

Plus préférentiellement, l'ecdystéroïde concernée est l'ecdystérone ou un analogue d'ecdystérone.

Les ecdystéroïdes, leurs analogues ou leurs dérivés sont obtenus sous forme isolée, ou sous forme d'un extrait à partir de toutes sources naturelles disponibles, ou encore par un procédé de synthèse chimique. Les principales sources naturelles d'ecdystéroïdes sont les insectes, les invertébrés marins et un grand nombre de plantes. Les ecdystéroïdes peuvent ainsi être extraits *d'Achyranthes bidentata, d'Achyranthes aspera,* de différentes espèces de *Paris,* de *Polypodium vulgare,* de *Cyanotis arachnoidea, d'Ajuga decumbens, d'Ajuga iva,* de *Pfaffia paniculata,* de *Pfaffia iresinoides,* de *Vitex glabrata,* de *Sesuvium portulacastrum,* de *Serratula sogdiana,* de *Serratula tinctoria,* de *Rhaponticum integrifolium,* de *Rhaponticum carthamoides,* de différentes espèces de *Silene,* de *Lychnis flos-cuculi,* de *Cyathula officinalis,* de *Cyathula capitata* ou de *Bombyx mori.* Plus particulièrement, parmi les plantes pouvant être utilisées pour l'extraction des ecdystéroïdes, en particulier de l'ecdystérone, on peut citer *Serratula sogdiana* et *Rhaponticum integrifolium,* qui présentent l'avantage de pouvoir être cultivées.

Par ailleurs un certain nombre d'ecdystéroïdes sont disponibles dans le commerce. L'ecdystérone est en particulier disponible chez Sigma-Aldrich sous la référence H5142.

La quantité en poids de d'ecdystéroïde, d'analogue d'ecdystéroïde ou de dérivé d'ecdystéroïde est comprise entre 0,001 et 10 % par rapport au poids total de la composition.

De préférence, la quantité en poids d'ecdystéroïde ou d'analogue d'ecdystéroïde ou de dérivé d'ecdystéroïde est comprise entre 0,01 et 5 % par rapport au poids total de la composition.

La composition cosmétique selon l'invention peut en outre comprendre d'autres agents cosmétiques destinés à favoriser la croissance pilaire comme par exemple l'aminexil (2,4 DPO, 2, 4 diamino pyrimidine-N-oxyde).

La composition selon l'invention peut comprendre en outre de l'eau, et/ou un ou des solvants organiques cosmétiquement acceptables. Ces solvants peuvent être choisis dans le groupe constitué par les solvants organiques hydrophiles, les solvants organiques lipophiles, les solvants amphiphiles ou leurs mélanges,

Parmi les solvants organiques hydrophiles, on peut citer par exemple des mono-alcools inférieurs, linéaires ou ramifiés, ayant de 1 à 8 atomes de carbone comme l'éthanol, le propanol, le butanol, l'isopropanol, l'isobutanol, des polyéthylèneglycols éventuellement oxyéthylénés, des polyols tels que le propylèneglycol, l'isoprèneglycol, le butylène glycol, le glycérol, le sorbitol et ses dérivés, les éthers de glycol et les éthers de propylène glycol.

Comme solvants organiques amphiphiles, on peut citer des polyols tels que des dérivés de propylèneglycol.

Comme solvants organiques lipophiles, on peut citer par exemple les esters gras.

La composition selon l'invention peut également comporter des adjuvants tels que des corps gras, des agents conservateurs, des agents stabilisants, des agents opacifiants, des adoucissants, des silicones, des agents moussants, des agents antioxydants, des agents régulateurs de pH, des agents émulsifiants, des gélifiants et/ou épaississants classiques hydropholes ou lipophiles, des actifs hydropholes ou lipophiles, des parfums, des émulsionnants, des séquestrants, des polymères, des agents acidifiants ou alcalinisants, des charges, des agents anti-radicaux libres, des céramides, des agents hydratants, des vitamines, des tensio-actifs, des antipelliculaires, des propulseurs, des colorants, ou tout autre adjuvant habituellement utilisé en cosmétique.

Les quantités de ces différents adjuvants sont celles classiquement utilisées dans le domaine considéré. L'homme de l'art veillera à choisir le ou les éventuels composés à ajouter à la composition selon l'invention de manière à ne pas ou substantiellement pas altérer les propriétés avantageuses attachées à la composition conforme à l'invention

Les compositions conformes à l'invention sont particulièrement appropriées sur le plan cosmétique et/ou dermatologique et ne provoquent pas d'irritation du cuir chevelu, même après un contact prolongé sans rinçage.

La composition selon l'invention peut se présenter sous toutes les formes galéniques normalement utilisées pour une application topique cosmétique ou dermatologique, notamment sous forme d'une solution aqueuse, hydroalcoolique ou huileuse, d'une émulsion huile-dans-eau ou eau-dans-huile ou multiple, d'un gel aqueux ou huileux.

La composition peut avoir l'aspect d'un lait, d'un gel, d'une mousse, d'un sérum ou d'une lotion.

Les compositions définies ci-dessus peuvent être appliquées sur les cheveux ou le cuir chevelu et peuvent s'appliquer, par exemple après un lavage du cuir chevelu.

Ces compositions sont destinées à être utilisées dans les cas de chute de cheveux que cette chute soit d'origine naturelle ou médicamenteuse c'est-à-dire suite à l'absorption de médicaments ayant pour effet secondaire une chute de cheveux).

La présente invention a également pour objet un procédé de traitement cosmétique du cuir chevelu dans le but d'empêcher une chute de cheveux, ce procédé consistant à appliquer sur les zones concernées, une quantité cosmétiquement efficace d'une composition contenant au moins un ecdystéroïde, un analogue ou un dérivé d'ecdystéroïde.

Les exemples suivants sont destinés à illustrer l'invention, sans toutefois présenter un caractère limitatif.

### Exemple 1 : Effet de l'ecdystérone sur la survie du follicule pileux humain in vitro.

Nous avions développé et breveté un modèle de follicule humain en survie *in vitro* (FR 95-08465) permettant d'évaluer l'effet d'une substance sur la survie et l'allongement du follicule.

Nous avons utilisé ce modèle *in vitro* pour évaluer l'effet de l'ecdystérone sur la croissance et la survie du follicule pileux. Différentes concentrations de cette substance ont été testées : 0,01 *µ*g/ml, 0,1 *µ*g/ml, 1*µ*g/ml et 10 *µ*g/ml.

Les tests statistiques effectués sur l'ensemble du prélèvement montrent que les différences observées entre les groupes sont significatives.

L'analyse statistique montre ainsi que l'ecdystérone a un effet positif sur la survie du follicule pileux humain *in vitro.* En effet, l'ecdystérone aux concentrations de 0,01 *µ*g/ml, 0,1 *µ*g/ml et 10 *µ*g/ml augmente de façon significative la survie du follicule pileux humain dans ces conditions de culture.

### Exemple 2 : Effets de l'ecdysterone sur le maintien de l'intégrité du follicule pileux in vitro

Afin de mieux caractériser l'effet de cette substance sur le follicule pileux, nous avons suivi l'activité mitotique des cellules du bulbe ainsi que les différents programmes de différenciation spécifiques de la gaine externe, de la gaine interne et de la tige pilaire, en présence ou en absence de cette substance.

L'intégrité des compartiments du follicule après 14 jours de culture a ainsi été suivie grâce à des anticorps reconnaissant des marqueurs spécifiques de chacun de ces compartiments : le marqueur de mitose Ki67 spécifique de la matrice (S. Commo & B.A. Bernard, 1997, *Br. J. Dermatol.***137** :31-38), la kératine K14 spécifique de la gaine externe (ibid.), la transglutaminase spécifique de la gaine interne (ibid.), et la kératine hHa5 spécifique de la tige pilaire et en particulier la zone kératogène et la cuticule) (L. Langbein et al., 1999, *J. Biol. Chem.* **274** :19874-19884).

Après 14 jours de culture, en absence d'ecdystérone, l'analyse histologique du follicule pileux monter que le marquage Ki67 diminue, la kératine K14 est exprimée de façon ectopique, l'expression de la transglutaminase signe un épaississement et une désorganisation de la gaine interne, et la kératine hHa5 montre une altération de la cuticule de la tige. Par contre, après 14 jours de culture en présence d'ecdysterone à 0.01 µg/ml, l'ensemble des marqueurs étudiés conserve une expression comparable à celle du follicule de référence à J=0.

L'ecdystérone s'oppose donc à la dégradation spontanée du follicule et maintient l'intégrité des compartiments folliculaires après 14 jours de *culture in vitro.*

### Exemple 3 : Composition cosmétique n°1 destinée à empêcher la chute de cheveux.

Les composants suivants sont pesés, mélangés et solubilisés à température ambiante.

| | |
|---|---|
| Ecdystérone | 9,0 |
| Polypropylène glycol | 5,0 |
| Vitamine E | 0,1 |
| Hydrochlorure de diphénhydramine | 0,1 |
| Glycyrrhétinate de dipotassium | 0,1 |
| Méthylparabène | 0,2 |
| ethanol | 50,0 |
| Parfum | 1,0 |

### Exemple 4 : Composition cosmétique n°2 destinée à empêcher la chute de cheveux.

Les composants suivants sont pesés, chauffés séparément et dissous à 80°C avant mélange.

| | |
|---|---|
| Ponastérone C | 0,5 |
| Inokostérone | 0,5 |
| Chlorure de stéaryl-méthyl ammonium | 3,5 |
| Polypropylène glycol | 5,0 |
| Paraffine liquide | 2,0 |
| Méthylpolysiloxane | 2,0 |
| Alcool béhénylique | 4,0 |
| Parfum | 0,5 |
| Eau purifiée | 82,0 |

## Revendications

1. Utilisation d'au moins un ecdystéroïde ou d'au moins un analogue ou d'au moins un dérivé d'ecdystéroïde pour la préparation d'une composition cosmétique ou dermatologique destinée à empêcher la chute de cheveux.

2. Utilisation selon la revendication 1, **caractérisée en ce que** l'ecdystéroïde, analogue d'ecdystéroïde ou dérivé d'ecdystéroïde est choisi parmi l'ecdystérone, l'ecdysone, la muristérone A, les ponastérones A, B et C, l'inokostérone, les limnanthéosides A et B, les ajugastérones B et C, la turkestérone, la dacryhainanstérone, la kaladastérone, la podécydsone, la stachystérone, ou les ecdystéroïdes 7, 9 (11)-dien-6-ones.

3. Utilisation selon la revendication 2, **caractérisée en ce que** l'ecdystéroïde est l'ecdystérone.

4. Utilisation selon la revendication 2, **caractérisée en ce que** l'ecdystéroïde est un analogue d'ecdystérone.

5. Utilisation selon la revendication 2, **caractérisée en ce que** l'ecdystéroïde est un dérivé d'ecdystérone.

6. Utilisation selon l'une des revendications 1 à 5, **caractérisée en ce que** la quantité en poids de l'ecdystéroïde ou de l'analogue de l'ecdystéroïde ou du dérivé d'ecdystéroïde est comprise entre 0,001 et 10 % par rapport au poids total de la composition finale.

7. Utilisation selon l'une des revendications 1 à 6, **caractérisée en ce que** la quantité en poids de l'ecdystéroïde ou de l'analogue de l'ecdystéroïde ou du dérivé d'ecdystéroïde est comprise entre 0,01 et 5 % par rapport au poids total de la composition finale.

8. Utilisation selon l'une quelconque des revendications 1 à 7 **caractérisée en ce que** la composition comprend de plus un agent cosmétique.

9. Utilisation selon la revendication 8, **caractérisée en ce que** la composition comprend du 2,4 DPO.

10. Utilisation selon l'une des revendications 1 à 9, **caractérisée par le fait qu'**elle comprend en plus, de l'eau, de l'alcool et un ou des solvants organiques cosmétiquement acceptables.

11. Utilisation selon l'une des revendications 1 à 10, **caractérisée par le fait qu'**elle comprend en plus, un ou des adjuvants cosmétiquement acceptables.

12. Utilisation selon l'une des revendications 1 à 11, **caractérisée par le fait qu'**elle se présente sous la forme d'une solution aqueuse, hydroalcoolique ou d'un gel aqueux ou hydroalcoolique.

13. Utilisation selon l'une des revendications 1 à 12, **caractérisée par le fait qu'**elle se présente sous la forme d'un lait, d'un gel, d'une mousse, d'un sérum ou d'une lotion.

14. Utilisation selon l'une quelconque des revendications 1 à 13, **caractérisée par le fait que** la composition cosmétique ou dermatologique est destinée à la prévention de chute de cheveux d'origine naturelle ou médicamenteuse.

15. Procédé de traitement cosmétique pour empêcher la chute de cheveux, **caractérisé en ce qu'**on applique sur les zones concernées du cuir chevelu d'une personne, une quantité cosmétiquement efficace d'une composition contenant au moins un ecdystéroïde ou un dérivé d'ecdystéroïde.
